# EUROPÄISCHE PATENTSCHRIFT

(11) **EP 2 878 331 B1**
(45) Veröffentlichungstag und Bekanntmachung des Hinweises auf die Patenterteilung: **21.09.2016**
(21) Anmeldenummer: 14190627.1
(22) Anmeldetag: 28.10.2014
(51) Int. Cl.: A61N 1/05

(54) **Elektrodenkopf mit zylinderschalensegmentförmigen Gehäuseteilen**
Electrode head with housing parts in the form of cylinder shell segments
Tête d'électrode avec une enveloppe présentant des parties en forme de segments d'une enveloppe de cylindre

(30) Priorität: 02.12.2013 US 201361910442 P
(43) Veröffentlichungstag der Anmeldung: 03.06.2015
(73) Patentinhaber: BIOTRONIK SE & Co. KG, 12359 Berlin (DE)
(72) Erfinder: Täubert, Kerstin, 12589 Berlin (DE); Hinrichsen, Lisa, 10999 Berlin (DE); Poplien, Patrick, 12459 Berlin (DE); Lenski, Hartmut, 15806 Zossen (DE); Günther, Thomas, 14552 Michendorf (DE)
(74) Vertreter: Galander, Marcus

(56) Entgegenhaltungen:
- US-A- 5 496 360
- US-A1- 2004 172 117
- US-A1- 2008 114 230
- US-A1- 2011 024 186
- US-A1- 2011 130 818

## Beschreibung

Die Erfindung betrifft einen Elektrodenkopf einer implantierbaren Elektrodenleitung, mit einem Kopfgehäuse. Sie betrifft des Weiteren eine Elektrodenleitung.

Implantierbare Elektrodenleitungen und zugehörige Elektrodenköpfe sind ein funktionswesentlicher Bestandteil verschiedenartiger medizin-elektronischer Anordnungen, speziell von Herzschrittmacher- und implantierbaren Defibrillatorsystemen, aber auch von Anordnungen zur Nerven- oder Gehirnstimulation sowie von Anordnungen zur Erfassung von Aktionspotentialen und anderen elektrischen Signalen aus dem Körper eines Patienten. Deren zuverlässige und für den Patienten in jedem Fall sichere Funktion ist daher unabdingbare Voraussetzung ihres klinischen Einsatzes. Auch Aspekte wie Sterilisierbarkeit, leichte Handhabung und vertretbare Kosten spielen bei der Entwicklung und dem Einsatz von Elektrodenleitungen und -köpfen eine wichtige Rolle.

Für viele Anwendungen müssen viele Elektrodenleitungen so gestaltet sein, dass ihr distales Ende am Bestimmungsort an oder in einem Hohlorgan (z.B. dem Herzen) oder Gefäß des Patienten sicher fixiert werden kann. Zur Erfüllung dieser Anforderung, unter Beachtung der oben genannten allgemeinen Anforderungen an Elektrodenleitungen, wurden über Jahrzehnte umfangreiche Entwicklungsbemühungen unternommen und die unterschiedlichsten technischen Lösungen vorgeschlagen.

Unter diesen haben 'Schraubköpfe' besondere praktische Bedeutung erlangt, bei denen im Elektrodenkopf eine Schraubwendel untergebracht ist, die im implantierten Zustand der Elektrodenleitung am Bestimmungsort vom Operateur aus dem Elektrodenkopf in distaler Richtung herausgedreht und dabei zugleich in den Gewebsabschnitt des Patienten eingeschraubt wird, an den der Elektrodenkopf distal anstößt. Bekannte Schraubköpfe bestehen aus mehreren miteinander verbundenen zylindrischen Hülsen, meist aus einem körperverträglichen Metall, gelegentlich aus Thermoplasten wie PU oder PEEK. Das Dokument US 2003/220676 A1 offenbart einen Elektrodenkopf mit distalen, einen Ring formenden Segmenten. Das Dokument WO 2009/023017 A1 beschreibt eine Elektrodenkopf mit gekrümmten, in einer Trägerstruktur aufgenommenen Elektroden.

Mit der Erfindung wird ein Elektrodenkopf bereitgestellt, der die Merkmale des Anspruchs 1 aufweist. Des Weiteren wird eine Elektrodenleitung mit einem derartigen Elektrodenkopf vorgeschlagen. Zweckmäßige Fortbildungen des Erfindungsgedankens sind Gegenstand der abhängigen Ansprüche.

Die Erfindung geht von der Überlegung aus, einen Elektrodenkopf (insbesondere Schraubkopf) zu entwickeln, der aus wenigen und preiswerten Einzelteilen besteht. Der Mechanismus ist dabei so zu gestalten, dass insbesondere ein Fixierungsmechanismus (speziell eine Schraube) in geeigneter geometrischer Konfiguration technologisch leicht realisierbar ist. Das aktuell praktizierte Aneinanderreihen von spritzgusstechnisch oder spanend hergestellten Einzelteilen führt immer zu einer großen Summentoleranz.

Ein wesentlicher Aspekt schalen besteht darin, das Gehäuse längs zu teilen. Dadurch entstehen zwei zylindersegmentförmige Gehäuseteile (Teilschalen), die derart ausgeformt sind, dass werkzeugseitig alle Anschläge, Zentrierungen und Steigungsgeber ausgeformt werden. Durch die Werkzeugtechnik wird gewährleistet, dass es zu keinen nennenswerten Maßabweichungen kommt. Nach dem Einlegen der Innenbaugruppe müssen die Teilschalen fest miteinander verbunden werden.

Der fertigungstechnische Aspekt, dass werkzeugseitig alle Anschläge, Zentrierungen und Steigungsgeber mit den Teilschalen mit ausgeformt werden, hat den wesentlichen Vorteil, dass die Teile auch in großer Stückzahl in höchster Wiederholgenauigkeit gefertigt werden können. Ein weiterer großer Vorteil besteht darin, dass diese Elemente immer den gleichen Abstand zueinander haben, weil diese Maße durch die Spritzform vorgegeben sind. Der Rand der Teilschalen ist dabei so gestaltet, dass diese sich sowohl in Längs- als auch in Querrichtung zentrieren.

Die aus derzeitiger Sicht bevorzugte Ausführung ist diejenige, bei der das Kopfgehäuse aus zwei in der Außenkontur halbzylindrischen Schalenteilen mit zur Längsachse paralleler Teilungsebene gefügt ist.

Des Weiteren sind zweckmäßigerweise die Gehäuseteile, insbesondere die halbzylindrischen Schalenteile, des Kopfgehäuses miteinander verschweißt oder verklebt.

In einer weiteren Ausführung sind insbesondere einige der Gehäuseteile Metallformteile, insbesondere Metallprägeteile aus dünnwandigem Metallblech. In einer weiteren Ausführung sind insbesondere einige der Gehäuseteile Kunststoffteile, speziell Spritzgussteile. Auch ein Kopfgehäuse aus einer Kombination von Metall- und Kunststoffteilen liegt im Bereich der Erfindung.

In einer weiteren Ausführung umfasst der Elektrodenkopf einen Dichtring und/oder einen Röntgenkontrastring, der/die in einer Ausführung in mindestens einer zweiten Kammer des Elektrodenkopfes aufgenommen ist/sind, die proximal von der ersten Kammer liegt. Der Röntgenkontrastring aus Metall kann gleichzeitig als umlaufende und geschlossene Dichtfläche für die Dichtung zwischen Welle und Gehäuse dienen.

In einer weiteren Ausführung weist der Elektrodenkopf in einem proximalen Abschnitt geringeren Außendurchmesser als in einem distal hiervon liegenden Abschnitt.

In einer weiteren Ausführung weist der Elektrodenkopf eine Mehrzahl von durch Querwandstücke abgegrenzten Kammern mit zentralem Durchgang auf. In einer zweckmäßigen Ausführung eines Schraubelektrodenkopfes sind drei abgegrenzte Kammern vorgesehen, von denen jede mindestens ein wesentliches Funktionselement der Schraubelektrode aufnimmt. Die innenliegende Baugruppe, im Normalfall bestehend aus Fixierungsschraube, Anschlaghülsen, Welle, Dichtung und Wendel kann komplett vormontiert werden. Notwendige Anschläge auf der Welle sind sichtbar und könnten mit höchster Genauigkeit zueinander lagefixiert werden. Auf diese Baugruppe wird anschließend der Röntgenring montiert. Die so komplettierte Baugruppe wird dann in die Unterschale eingelegt, die Oberschale montiert und abschließend werden beide Halbschalen miteinander verbunden.

In einer Ausführung der vorgeschlagenen Elektrodenleitung ist der Elektrodenkopf mittels mindestens eines Verbindungsschlauchstückes an den Elektrodenleitungskörper angefügt.

In einer weiteren Ausführung der vorgeschlagenen Elektrodenleitung ist der Elektrodenkopf mittels mindestens eines elastischen Verbindungsschlauchstückes an den Elektrodenleitungskörper angefügt.

In weiteren, praktisch besonders bedeutsamen Ausführungen umfasst die Elektrodenleitung ein aktives Fixierungselement, welches insbesondere in einer distal liegenden ersten Kammer des Elektrodenkopfes aufgenommen ist. Speziell ist das Fixierungselement als drehbare und verschiebliche Schraubwendel ausgebildet. Alternativ kann es sich aber auch um verschiebliche und aufspreizbare Klauen, bewegliche Finnen oder andere an sich bekannte Fixierungselemente halten, deren Realisierung mit dem vorgeschlagenen Aufbau des Kopfgehäuses kompatibel ist und das Vorsehen eines Betätigungs- bzw. Kraftübertragungselementes (z. B. einer Schraubwendel-Betätigungswelle) innerhalb des Leitungskörpers der Elektrodenleitung erfordert.

Vorteile und Zweckmäßigkeiten des Elektrodenkopfes ergeben sich im Übrigen aus der nachfolgenden Beschreibung eines Beispiels anhand der Figuren. Von diesen zeigen:
- Fig. 1: eine perspektivische Außenansicht eines Elektrodenkopfes
- Fig. 2A und 2B: eine perspektivische Ansicht des Innenaufbaus (Fig. 2A) bzw. der Innenwandung einer Halbschale des Elektrodenkopfes aus Fig. 1 und
- Fig. 3: eine Längsschnitt Darstellung des Aufbaus einer mit dem Elektrodenkopf nach Fig. 1 bis 2B versehenen Elektrodenleitung (distaler Teil).

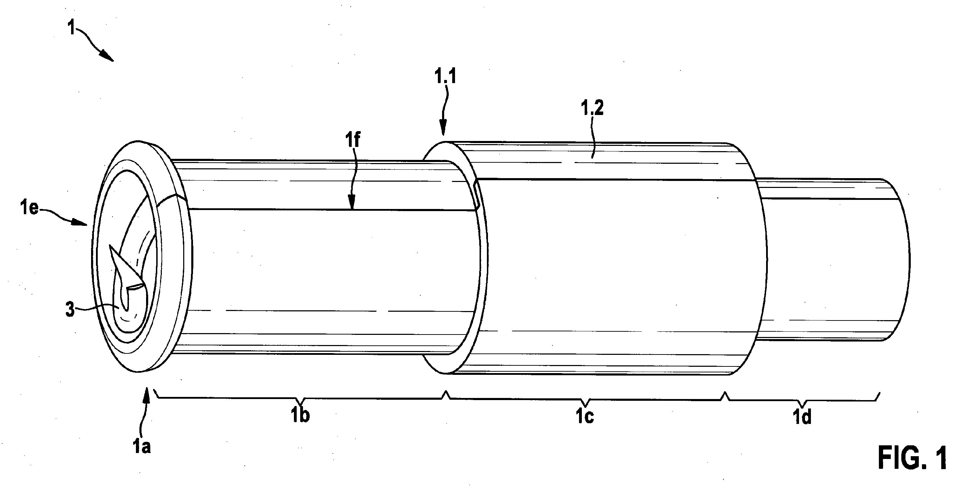

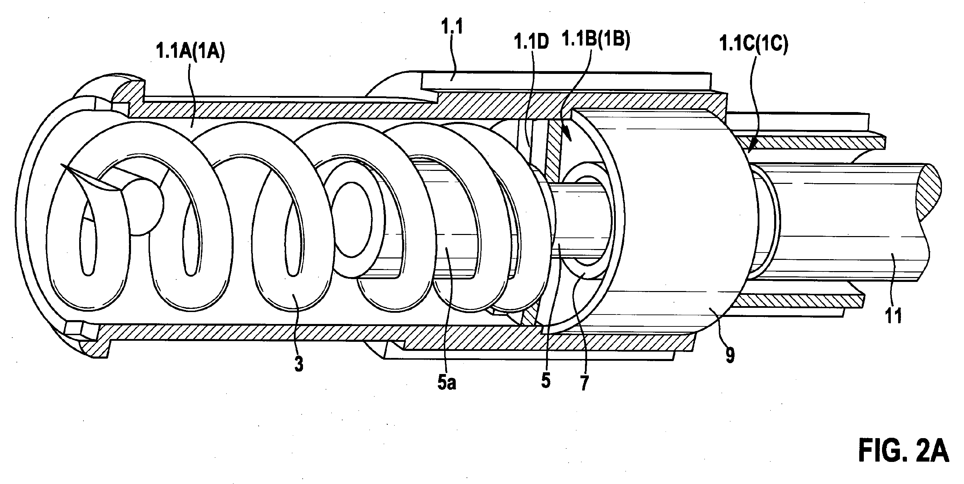

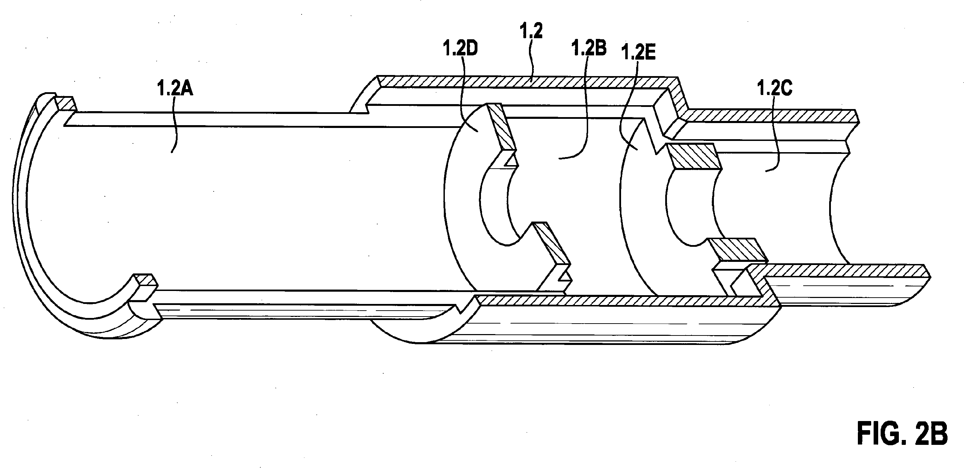

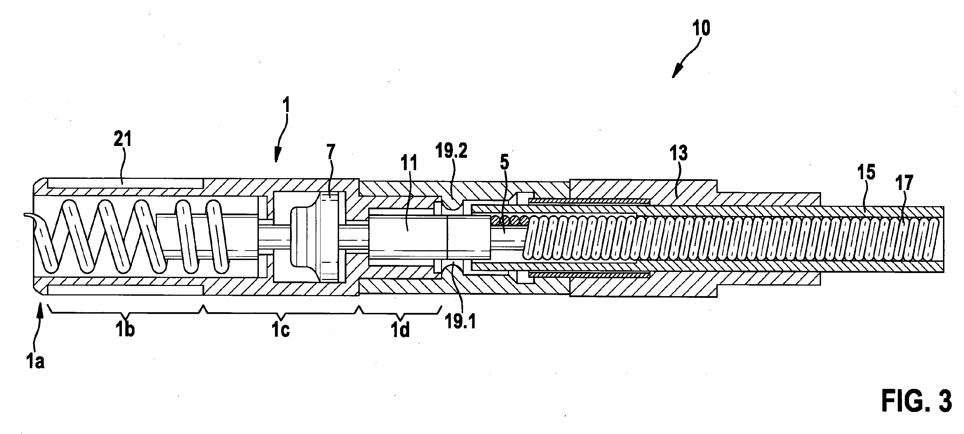

Fig. 1 zeigt in perspektivischer Darstellung einen in seiner Grundform zylindrischen Schraubelektrodenkopf 1 einer implantierbaren Schrittmacher-Elektrodenleitung (siehe Fig. 3), dessen Konstruktion in Fig. 2A und 2B genauer gezeigt ist. Am distalen Ende hat der Elektrodenkopf 1 einen abgerundeten Flanschabschnitt 1a eines ersten Durchmessers, an den sich (in dieser Reihenfolge) ein erster längerer Zylinderabschnitt 1b geringeren Durchmessers, ein zweiter längerer Zylinderabschnitt 1c größeren Durchmessers und schließlich ein dritter längerer Zylinderabschnitt 1d mit wieder verringertem und auch unterhalb des Durchmessers des ersten Zylinderabschnitts 1b liegenden Durchmessers anschließen. Überdies ist zu erkennen, dass der Elektrodenkopf 1 ein offenes distales Ende 1e hat, in dem das distale Ende einer Schraubwendel 3 zu sehen ist. Zu erkennen ist weiterhin eine durchgehende Schweißnaht 1f, die vorgefertigte (hier nicht separat bezeichnete) Halbschalen 1.1 und 1.2 des Elektrodenkopfes 1 stoffschlüssig fest miteinander verbindet.

Fig. 2A zeigt den Innenaufbau des Elektrodenkopfes 1, zusammen mit der ersten Halbschale 1.1 des Kopfgehäuses, und Fig. 2B zeigt die zweite Halbschale 1.2 des Kopfgehäuses, jeweils wiederum in perspektivischer Darstellung. Der besseren Übersichtlichkeit halber sind die weiter oben genannten Abschnitte 1a bis 1e der Außenkontur des Kopfgehäuses in Fig. 2A und 2B nicht nochmals bezeichnet. Es ist jedoch zu erkennen, dass beide Halbschalen 1.1, 1.2 eine (natürlich ebenfalls halbschalige) Unterteilung in drei in Längsrichtung aneinander anschließende Kammersegmente 1.1A, 1.1B und 1.1C bzw. 1.2A, 1.2B und 1.2C aufweisen. Die genannten Kammersegmente sind durch prinzipiell kreissegmentförmige Trennwände mit zentralem Durchgang voneinander abgetrennt, von denen in Fig. 2A lediglich die distalere Trennwand 1.1D der ersten Halbschale 1.1, in Fig. 2B jedoch beide Trennwände 1.2D und 1.2E zu erkennen sind.

Im Vergleich der beiden Figuren ist gut zu erkennen, dass die Halbschalen 1.1, 1.2 nicht identisch sind, sondern unterschiedliche Vor- und Rücksprünge bzw. Anschläge und Zentrierungen sowohl im Bereich des distalen Flansches als auch von zwei der drei abgestuften zylindrischen Bereiche und schließlich auch der Trennwände aufweisen. Unter anderem wird hierdurch gewährleistet, dass es in der Wandung des Kopfgehäuses hinreichend große Überlappungsbereiche zwischen der ersten und zweiten Halbschale zur Gewährleistung hoher mechanischer Stabilität und Dichtigkeit des hinteren Bereiches des Kopfgehäuses gibt.

In Fig. 2A ist zu erkennen, wie die Schraubwendel 3 die distale Kammer 1A des Elektrodenkopfes 1 im zurückgezogenen Zustand in ihrer ganzen Länge ausfüllt und mit ihrem proximalen Ende einen Stößel 5a einer Fixierungsschrauben-Betätigungswelle 5 umschließt, an dem sie (was hier nicht zu erkennen ist) stoffschlüssig befestigt ist (etwa durch Verschweißen). In der zweiten Kammer 1B ist die Fixierungsschrauben-Betätigungswelle 5 von einem Dichtring 7 und einem Röntgenkontrastring 9 derart umgeben, dass der Umfang der Kammer weitgehend ausgefüllt ist. In der dritten Kammer 1c ist eine Kontakthülse 11 zur elektrischen Kontaktierung der (hier nicht gezeigten) Leiterwendel des Elektrodenleitungskörpers mit dem Stößel 5a bzw. der Schraubwendel 3 zu sehen.

Fig. 3 zeigt in einer Längsschnittdarstellung den distalen Endbereich einer mit dem Elektrodenkopf 1 nach Fig. 1 bis 2B versehenen Elektrodenleitung 10. Neben dem Elektrodenkopf 1, dessen äußere Gestalt und Aufbau in Fig. 1 bis 2B gezeigt und weiter oben beschrieben sind, umfasst die Elektrodenleitung 10 einen (hier nicht gezeigten) äußeren Leitungskörper oder Außenschlauch, eine zur Kontaktierung einer Ringelektrode 13 dienende und hier ebenfalls nicht dargestellte Außenleiter-Wendel und eine innerhalb eines Innenschlauches 15 angeordnete Innenleiter-Wendel 17. In deren Innerem wiederum ist die bereits erwähnte Fixierungsschrauben-Betätigungswelle 5 platziert und an ihrem Ende ist die ebenfalls bereits weiter oben erwähnte Kontakthülse 11 angebracht.

Die mechanische Verbindung und zugleich Abdichtung zwischen dem Elektrodenkopf 1 und dem Leitungskörper der Leitung 10 distal von der Ringelektrode 13 ist durch einen innen liegenden PTFE-Schlauch 19.1 und ein im Wesentlichen ebenfalls schlauchförmiges äußeres Silikon-Verbindungsstück 19.2 bewerkstelligt. Während der PTFE-Schlauch 19.1 innerhalb der proximalen Kammer 1C des Elektrodenkopfes 1 liegt und sich bis über einen distalen Bereich der Innenleiter-Wendel 17 erstreckt, umgreift das Silikon-Verbindungsstück 19.2 einen distalen Endabschnitt der Ringelektrode 13 ebenso wie die Außenwandung der proximalen Kammer 1C (oder, bezogen auf die Außenansicht, den proximalen Zylinderabschnitt 1d) des Elektrodenkopfes 1 und verbindet diese miteinander. Geeignet ausgeformte Fixierungsring- bzw. Arretierungsabschnitte in der Innenwandung des Silikon-Verbindungsstücks 19.2 bzw. der Außenwandung des PTFE-Schlauches 19.1 und ggfs. zusätzliche Fixierungsringe oder andere Fixierungsmittel tragen zur sicheren Fixierung der miteinander verbundenen Teile in Elektrodenleitungs-Längsrichtung in an sich bekannter Weise bei.

Besonders hinzuweisen ist in Fig. 3 noch auf die vollständige Darstellung des Dichtringes 7, von dem in Fig. 2A nur ein distaler Stirnseitenabschnitt zu sehen ist. Es ist zu erkennen, dass die Außenkontur des Dichtringes 7 mehrfach abgestuft bzw. abgeschrägt ist. Des Weiteren ist hier im Bereich des distalen Zylinderabschnitts 1B des Elektrodenkopfes 1 ein hohlzylindrischer Medikamentenspeicherbereich 21 dargestellt, der (wiederum in an sich bekannter Weise) zur Speicherung und langfristig kontinuierlichen Abgabe eines entzündungshemmenden Steroids im implantierten Zustand der Elektrodenleitung dient. Wenn die Halbschalen schon fest zum zylindrischen Elektrodenkopf 1 verbunden sind, wird der Medikamentenspeicher in Form eines Hohlzylinders 21 im distalen Bereich übergeschoben und befestigt.

Bei dem gezeigten Beispiel ist der Elektrodenkopf 1 bevorzugt aus PEEK-Halbschalen gefertigt, alternativ können aber auch PU-Halbschalen oder Gehäuseteile aus einem anderen in implantierten Teilen einsetzbaren Polymer gefertigt werden. Grundsätzlich kommt auch die Fertigung aus einem biokompatiblen Metall, etwa Titan oder einer Titanlegierung oder Edelstahl, in Betracht, wobei allerdings besondere konstruktive Vorkehrung zur Gewährleistung der Röntgensichtbarkeit der Ausschraublänge der Schraubwendel getroffen und ggfs. spezielle Federbereiche vorgesehen werden müssen, die in der oben beschriebenen Ausführung nicht vorhanden sind.

## Patentansprüche

1. Elektrodenkopf (1), proximal mit einer implantierbaren Elektrodenleitungskörper (10) verbindbar, distal zur Fixierung an oder in einem Hohlorgan oder Gefäß ausgebildet, mit einem langgestreckten und eine Längsachse aufweisenden zylindrischen Kopfgehäuse, wobei das Kopfgehäuse mindestens zwei zylinderschalensegmentförmige Gehäuseteile (1.1, 1.2) aufweist, die fest zusammengefügt sind, **dadurch gekennzeichnet, dass** die Gehäuseteile die Zylinderschale des Kopfgehäuses bilden.

2. Elektrodenkopf (1) nach Anspruch 1, wobei das Kopfgehäuse aus zwei in der Außenkontur halbzylindrischen Schalenteilen (1.1, 1.2) mit zur Längsachse paralleler Teilungsebene gefügt ist.

3. Elektrodenkopf (1) nach Anspruch 1 oder 2, wobei die Gehäuseteile, (1.1, 1.2) insbesondere die halbzylindrischen Schalenteile, des Kopfgehäuses miteinander verschweißt (1f) oder verklebt sind.

4. Elektrodenkopf (1) nach einem der vorangehenden Ansprüche, wobei die Gehäuseteile Metallformteile, insbesondere Metallprägeteile, sind.

5. Elektrodenkopf (1) nach einem der Ansprüche 1 bis 3, wobei die Gehäuseteile (1.1, 1.2) Kunststoffteile, insbesondere Spritzgussteile aus PEEK, sind.

6. Elektrodenkopf (1) nach einem der vorangehenden Ansprüche, wobei der Elektrodenkopf nem proximalen Abschnitt geringeren Außendurchmesser als in einem distal hiervon liegenden Abschnitt, aufweist.

7. Elektrodenkopf (1) nach einem der vorangehenden Ansprüche, mit einem offenen distalen (1e) und/oder proximalen Ende.

8. Elektrodenkopf (1) nach einem der vorangehenden Ansprüche, der eine Mehrzahl von durch Querwandstücke abgegrenzten Kammern (1A bis 1c) mit zentralem Durchgang aufweist.

9. Elektrodenkopf (1) nach einem der vorangehenden Ansprüche, mit einem Medikamentenspeicherbereich (21) in einem Abschnitt der Außenoberfläche.

10. Elektrodenkopf (1) nach Anspruch 9, wobei der Medikamentenspeicherbereich (21) ringförmig ist.

11. Elektrodenleitung (10) mit einem langgestreckten biegsamen Elektrodenleitungskörper und einem an den Elektrodenleitungskörper distal angefügten Elektrodenkopf (1) nach einem der vorangehenden Ansprüche.

12. Elektrodenleitung (10) nach Anspruch 11, wobei der Elektrodenkopf (1) mittels mindestens eines elastischen Verbindungsschlauchstückes (19.1, 19.2) an den Elektrodenleitungskörper angefügt ist.

13. Elektrodenleitung (10) nach Anspruch 11 oder 12, mit einem aktiven Fixierungselement (3), welches insbesondere in einer distal liegenden ersten Kammer (1A) des Elektrodenkopfes (1) aufgenommen ist.

14. Elektrodenleitung (10) nach Anspruch 13, wobei das Fixierungselement (3) als drehbare und verschiebliche Schraubwendel ausgebildet ist.

15. Elektrodenleitung (10) nach einem der Ansprüche 11 bis 14, mit einem Dichtring (7) und/oder einem Röntgenkontrastring (9), der/die insbesondere in mindestens einer zweiten Kammer (1B) des Elektrodenkopfes (1) aufgenommen ist/sind, die proximal von der ersten Kammer (1A) liegt.

## Claims

1. An electrode head (1) which can be connected at a proximal end to an implantable electrode lead body (10) and at a distal end is designed for fixing to or in a hollow organ or vessel, comprising an elongate cylindrical head housing which has a longitudinal axis, wherein the head housing has at least two housing parts (1.1, 1.2), which are shaped in the form of cylinder shell segments and are fixedly joined together, **characterised in that** the housing parts form the cylinder shell of the head housing.

2. The electrode head (1) according to claim 1, wherein the head housing is joined from two shell parts (1.1, 1.2) which have a semi-cylindrical outer contour and a dividing plane parallel to the longitudinal axis.

3. The electrode head (1) according to claim 1 or 2, wherein the housing parts (1.1, 1.2), in particular the semi-cylindrical shell parts, of the head housing are welded (1f) or adhesively bonded to one another.

4. The electrode head (1) according to any one of the preceding claims, wherein the housing parts are metal shaped parts, in particular metal stamped parts.

5. The electrode head (1) according to any one of claims 1 to 3, wherein the housing parts (1.1, 1.2) are plastic parts, in particular injection-moulded parts made of PEEK.

6. The electrode head (1) according to any one of the preceding claims, wherein the electrode head, in a proximal portion, has an outer diameter smaller than in a portion arranged distally in relation hereto.

7. The electrode head (1) according to any one of the preceding claims, comprising an open distal (1e) and/or proximal end.

8. The electrode head (1) according to any one of the preceding claims, the electrode head having a plurality of chambers (1A to 1c) which are delimited by transverse wall pieces and which have a central aperture.

9. The electrode head (1) according to any one of the preceding claims, comprising a drug storage region (21) in a portion of the outer surface.

10. The electrode head (1) according to claim 9, wherein the drug storage region (21) is annular.

11. An electrode lead (10) having an elongate flexible electrode lead body and an electrode head (1) according to any one of the preceding claims attached distally to the electrode lead body.

12. The electrode lead (10) according to claim 11, wherein the electrode head (1) is attached by means of at least one resilient connection tube piece (19.1, 19.2) to the electrode lead body.

13. The electrode lead (10) according to claim 11 or 12, comprising an active fixing element (3) which is received in particular in a distally arranged first chamber (1A) of the electrode head (1).

14. The electrode lead (10) according to claim 13, wherein the fixing element (3) is formed as a rotatable and displaceable helical coil.

15. The electrode lead (10) according to any one of claims 11 to 14, comprising a ring seal (7) and/or an X-ray contrast ring (9) which are/is arranged in at least one second chamber (1B) of the electrode head (1) arranged proximally in relation to the first chamber (1A).

## Revendications

1. Tête d'électrode (1), pouvant être reliée de manière proximale avec un corps de ligne d'électrode (10) implantable, conçu de manière distale pour la fixation sur, ou dans, un organe creux ou un vaisseau, avec un boîtier de tête cylindrique allongé et présentant un axe longitudinal, où le boîtier de tête présente au moins deux parties de boîtier (1.1, 1.2) en forme de segments de corps de cylindre, qui sont solidement assemblées, **caractérisée en ce que** les parties de boîtier forment le corps de cylindre du boîtier de tête.

2. Tête d'électrode (1) selon la revendication 1, où le boîtier de tête est accommodé dans son contour extérieur de deux parties de corps (1.1, 1.2) demi cylindriques avec un plan de séparation parallèle à l'axe longitudinal.

3. Tête d'électrode (1) selon la revendication 1, ou 2, où les parties de boîtier (1.1, 1.2), notamment les parties de corps demi cylindriques, du boîtier de tête sont soudées ensemble (1f) ou collées.

4. Tête d'électrode (1) selon l'une des revendications précédentes, où les parties de boîtier sont des parties de moule métallique, notamment des parties d'estampage de métal.

5. Tête d'électrode (1) selon l'une des revendications 1 à 3, où les parties de boîtier (1.1, 1.2) sont des parties en matière plastique, notamment des parties moulées par injection en polyétheréthercétone PEEK.

6. Tête d'électrode (1) selon l'une des revendications précédentes, où la tête d'électrode présente un diamètre extérieur dans une section proximale inférieur à celui dans une section se situant de manière distale par rapport à celle-ci.

7. Tête d'électrode (1) selon l'une des revendications précédentes, avec une extrémité distale (1e) et/ou proximale ouverte(s).

8. Tête d'électrode (1) selon l'une des revendications précédentes, qui présente une multitude de chambres (1A à 1c) délimitées par des morceaux de parois transversales avec un passage central.

9. Tête d'électrode (1) selon l'une des revendications précédentes, avec une région de stockage de médicaments (21) dans une portion de la surface extérieure.

10. Tête d'électrode (1) selon la revendication 9, où la région de stockage de médicaments (21) est de forme annulaire.

11. Ligne d'électrode (10) avec un corps de ligne d'électrode allongé pliable et une tête d'électrode (1) selon l'une des revendications précédentes accommodée de manière distale sur le corps de ligne d'électrode.

12. Ligne d'électrode (10) selon la revendication 11, où la tête d'électrode (1) est fixée sur le corps de ligne d'électrode au moyen d'au moins une pièce de tuyau de liaison (19.1, 19.2) élastique.

13. Ligne d'électrode (10) selon la revendication 11, ou 12, avec un élément de fixation (3) actif, lequel est notamment logé dans une première chambre (1A) de la tête d'électrode (1) se situant de manière distale.

14. Ligne d'électrode (10) selon la revendication 13, où l'élément de fixation est conçu sous la forme d'une hélice pouvant être mise en rotation et être déplacée.

15. Ligne d'électrode (10) selon l'une des revendications 11 à 14, avec un anneau d'étanchéité (7) et/ou un anneau de contraste pour rayons X (9), qui est /sont notamment logé /logés dans au moins une deuxième chambre (IB) de la tête d'électrode (1), qui se situe de manière proximale par rapport à la première chambre.
